# EUROPEAN PATENT APPLICATION

(11) **EP 4 111 977 A1**
(43) Date of publication of application: **04.01.2023**
(21) Application number: 21182370.3
(22) Date of filing: 29.06.2021
(51) Int. Cl.: A61B 6/00, A61B 5/08, G01N 23/041, A61B 5/00

(54) **A METHOD FOR X-RAY IMAGE PROCESSING**

(71) Applicant: Koninklijke Philips N.V., 5656 AG Eindhoven (NL)
(72) Inventor: VON BERG, Jens, 5656 AE Eindhoven (NL); KOEHLER, Thomas, 5656 AE Eindhoven (NL)
(74) Representative: Philips Intellectual Property & Standards

(57) **Abstract**

Concepts for generating a dark X-ray (DAX) model adapted to represent relations between a DAX signal, a respiratory state, and a respiratory disease grade, as well as concepts for using the DAX model are proposed. In particular, as relations between these variables are modelled, the difference between a DAX signal of a subject at a respiratory state, compared to a DAX signal of a subject at a reference respiratory state may be assessed. Thus, the diagnostic accuracy of DAX imaging may be improved.

## Description

### FIELD OF THE INVENTION

The invention relates to the field of dark-field X-ray imaging, and more specifically to the field of processing dark-field X-ray images.

### BACKGROUND OF THE INVENTION

In conventional techniques of X-ray imaging, an object is exposed to X-rays to obtain a transmission image of the object. However, for soft tissues including vessels, cartilages, lungs, and breast tissues with little absorption, this provides poor attenuation contrast compared with bone images. In order to overcome this limitation, there have been major developments in recent years to obtain dark-field X-ray (DAX) images using X-rays.

One of the primary differences between DAX and conventional X-ray lies in the X-ray energy. In regular transmission images of human patients an X-ray energy of about 125 kVp is used, as the ribs have less contrast for high kVp. However, DAX imaging is more sensitive at lower X-ray energies, with X-ray energies of about 70 kVp being used.

DAX imaging has recently demonstrated an improved diagnostic accuracy for the diagnosing and staging of pulmonary disorders, such as emphysema, chronic obstructive pulmonary disease (COPD), lung fibrosis, pneumonia and lung cancer. This is because the DAX signal provides complementary information on microstructural properties in lung parenchyma that would be otherwise unaccessible from conventional X-ray imaging (e.g. X-ray and X-ray computed tomography). By way of example, DAX imaging is not only capable of capturing X-ray transmission but also small angle scatter, which occurs in healthy lung parenchyma but not in emphysematic lung areas.

In addition, it has recently been discovered that the inhalation state of the patient has an impact on the DAX signal strength. It is likely that this is due to changes for example in the size and/or density of the alveolars, or the thickness of their walls, during respiration. Chest X-rays are typically taken while the subject holds their breath - either at full inhalation or at full exhalation state. However, patients suffering from respiratory diseases, especially for example COPD and emphysema, are often not capable of breathing as requested for a standard acquisition procedure. This poses a significant problem in the accurate diagnosis and grading of pulmonary disorders using DAX imaging.

### SUMMARY OF THE INVENTION

The invention is defined by the claims.

According to examples in accordance with an aspect of the invention, there is provided a method of generating a dark-field X-ray, DAX, model adapted to represent relations between a DAX signal, a respiratory disease grade, and a respiration state. The method comprises: inputting a plurality of training DAX images of subjects at a plurality of known respiratory states, and with a plurality of known respiratory disease grades to the DAX model; receiving a plurality of predicted respiratory states and predicted respiratory disease grades from the DAX model; and determining parameter values of the DAX model based on a difference between the plurality of known respiratory states and the plurality of predicted respiratory states, and further based on a difference between the plurality of known respiratory disease grades and the plurality of predicted respiratory disease states.

In this way, proposed embodiments may provide a DAX model which calculates or predicts changes in a DAX signal depending on a respiration state. Accordingly, changes in a DAX signal acquired from a subject at a respiration state which deviates from a desired respiration state may be compensated for. In other words, the impact of a respiration state on the DAX signal may be assessed and accounted for (based on one or more preictions provided by the model).

Further, embodiments may provide a DAX model that represents an impact of a respiratory disease grade on the DAX signal. Thus, a more accurate model may be acquired as a respiratory disease grade may have an impact on the difference in DAX signal caused by a change in respiration state.

The DAX model may be trained using a number of DAX training images. This may be achieved by comparing a difference between a respiration state determined by the model, and an actual respiration state corresponding to the DAX image. Parameter values of the model may then be determined by this difference. In addition, a difference between a respiratory disease grade determined by the model, and an actual respiratory disease grade corresponding to the DAX image can be determined, which may further refine the parameter values.

By basing the DAX model on a large number of DAX training images, the parameter values may provide a more accurate representation of the interations between an unseen DAX signal, a respiratory disease grade, and a respiration state.

In some embodiments, the DAX model may use a statistical method to represent the relations between the DAX signal, the respiratory disease grade, and the respiration state.

In some embodiments, the DAX model may be further adapted to represent relations between the DAX signal, the respiratory disease grade, the respiration state and one or more test subject specific variables. In this case, the plurality of training DAX images may further comprise training DAX images of subjects with the one or more test subject specific variables. The DAX model may employ a statistical method to represent the relations between the DAX signal, the respiratory disease grade, the respiration state and the one or more test subject specific variables.

It has been realized that subject-specific variables, such as sex or age, may also affect the relations between the DAX signal and the respiratory state. By adapting the model to also represent these relations, a more accurate model of relations may be provided. By way of example, the one or more test subject specific variables may include a sex, a body size, a body mass index, a lung function test result, a respiratory disease rating, known diseases, or a smoking history.

In some embodiments, the DAX signal may be either an integrated signal over a full lung field of a DAX image, or an integrated signal over part of the full lung field of the DAX image.

According to another aspect of the invention, there is provided a method for processing a dark-field X-ray, DAX, signal of a subject. The method comprises generating a DAX model according to an aspect of the invention; obtaining the DAX signal of the subject, where a DAX image corresponds to the DAX signal; determining a respiratory state of the subject based on the DAX image; and acquiring a modified DAX signal of the subject at a reference respiration state based on inputting the DAX signal and the determined respiratory state to the generated DAX model.

In this way, a DAX model according to a proposed embodiment may be used to acquire a modified DAX signal. A respiratory state can be determined, and thereby the DAX model may output a modified DAX signal corresponding to a reference respiratory state. In other words, a difference betweeen a DAX signal of the subject at a respiratory state during acquisition, and a DAX signal of the subject at a reference respiratory state may be determined. Thus, improved accuracy of diagnosis and determination of a respiratory disease grade may be achieved.

According to yet another aspect of the invention, there is provided a method for processing a dark-field X-ray, DAX, signal of a subject using a DAX model adapted to represent relations between a DAX signal, a respiratory disease grade and a respiration state. The method comprises obtaining a DAX signal of the subject, where a DAX image corresponds to the DAX signal; determining a respiratory state of the subject based on the DAX signal; and acquiring a modified DAX signal of the subject at a reference respiration state based on inputting the DAX signal and the determined respiratory state to the DAX model.

In some embodiments, the method for processing a dark-field X-ray, DAX signal may further comprise obtaining a respiratory disease grade of the subject, and acquiring the modified DAX signal of the subject at the reference respiration state may be further based on inputting the obtained respiratory disease grade to the DAX model.

By ascertaining the respiratory disease grade of the subject, a modified DAX signal of the subject at the reference respiration state may be more accurately output from the DAX model.

In some embodiments, the DAX model may be further adapted to represent relations between the DAX signal, the respiratory disease grade, the respiration state and one or more subject specific variables. Also, the method may further comprise obtaining subject specific variable values of the subject, and acquiring the modified DAX signal of the subject at the reference respiration state may be further based on inputting the obtained subject specific variable values to the DAX model.

In some embodiments, the method may further comprise acquiring a predicted respiratory disease grade of the subject based on inputting the DAX signal and the determined respiratory state to the DAX model. Accordingly, the DAX model may be utilized to perform prediction of a respiratory disease grade of a subject.

In some embodiments, the reference respiration state may be either a full inhalation state, or a full exhalation state.

In some embodiments, determining the respiratory state of the subject may comprise estimating a location of a diaphragm of the subject in the DAX image, relative to ribs of the subject in the DAX image; and determining the respiratory state of the subject is based on the estimated location of the diaphragm. By determining the repsiratory state of the subejct by the acquired DAX image, a need for additional monitoring apparatus may be obviated.

In some embodiments, determining the respiratory state of the subject may comprise estimating a volume of air in a thorax of the subject based on a lung field of the DAX image; and determining the respiratory state of the subject based on the estimated volume of air in the thorax of the subject.

In some embodiments, the method may further comprise modifying the DAX image based on the modified DAX signal.

In another aspect of the invention, there is provided a computer program element, which, when being executed by at least one processing unit, is adapted to cause the processing unit to perform the steps of of any of the methods previously described.

In another aspect there is provided a computer readable medium having stored the program element.

According to another aspect of the invention, there is provided a system for processing a dark-field X-ray, DAX, signal of a subject. The system comprises a modelling unit configured to generate a DAX model adapted to represent relations between a DAX signal, a respiratory disease grade and a respiration state; an x-ray image acquisition unit configured to acquire the DAX signal of the subject wherein a DAX image corresponds to the DAX signal; a respiratory state unit configured to determine a respiratory state of the subject based on the DAX image; and a signal modification unit configured to acquire a modified DAX signal of the subject at a reference respiration state based on inputting the DAX signal and the determined respiratory state to the generated DAX model.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a flow diagram of a method of generating a DAX model;
Fig. 2 is a flow diagram of a method of processing a DAX signal of a subject including generating a DAX model;
Fig. 3 is a flow diagram of a method of processing a DAX signal of a subject by using a DAX model according to an embodiment;
Fig. 4 is a flow diagram of a method of processing a DAX signal of a subject by using a DAX model, and additional data;
Fig. 5A is a flow diagram of a method of determining a respiratory state of a subject;
Fig. 5B is a flow diagram of an alternative method of determining a respiratory state of a subject;
Fig. 6 is a block diagram representing a system for processing a DAX signal of a subject.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to".

It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes and/or solutions pertaining to a dark field X-ray (DAX) model representing the relations between a DAX signal, a respiratory state, and a respiratory disease grade. Some examples of respiratory diseases are emphysema, chronic obstructive pulmonary disease (COPD), lung fibrosis, pneumonia, COVID-19, and lung cancer. According to proposed concepts, a number of possible solutions may be implemented separately or jointly. That is, although these possible solutions may be described below separately, two or more of these possible solutions may be implemented in one combination or another.

Embodiments of the invention aim to provide a DAX model capable of representing relations between a DAX signal, a respiratory state, and a respiratory disease grade. In this way, changes in the DAX signal due to a deviation in the respiratory state may be ascertained. Further, the impact of a respiratory disease grade on the changes in the DAX signal due to a deviation in the respiratory state may be understood.

Such a DAX model may be used in some embodiments in order to modify a DAX signal of a subject, such that the modified DAX signal corresponds to a subject at a reference respiratory state. Thus, embodiments may improve the accuracy of diagnosis of pulmonary disorders in a subject.

By way of explanation, DAX promises to be sensitive to emphysema detection more than other medical imaging techniques, such as conventional X-ray computed tomography (CT). This is because DAX is not only capable of capturing x-ray transmission, but also small angle scatter that occurs in healthy lung parenchyma but not in emphysematic lung areas. However, the inhalation state of an imaged subject has an impact on the DAX signal strength.

To stage the grade of a respiratory disorder it is beneficial to control all other factors that define the DAX signal strength, and it is proposed that the respiration state is one of the most impactful factors.

Accordingly, X-rays of the lungs are normally acquired while the subject holds their breath either at a full inhalation or at a full exhalation state. For example, it is often the case that patients suffering from respiratory diseases, as well as those who are particularly young or old, do not (or cannot) breath as required for a standard acquisition procedure.

It is proposed that estimating an actual respiratory status of a subject during image acquisition would help compensating for the impact it has compared to the desired protocol respiratory state during the acquisition.

In particular, proposed embodiments may provide the following:
(i) Modelling of the impact of a respiration state on a DAX signal strength;
(ii) Estimation of the respiration state of an acquired DAX signal; and
(iii) Compensation of the impact of the deviation of the respiratory state from a reference state on the DAX signal.

Through embodiments providing modified measurements, the diagnostic potential of DAX imagining for respiratory disease quantification and assessment may be improved. Such modified measurements may be capable of improved comparability between subjects and also within a subject in a longitudinal examination. Further, according to some embodiments, quantitative measurements may be corrected, and images provided to human readers may be modified to better reflect the modified measurement.

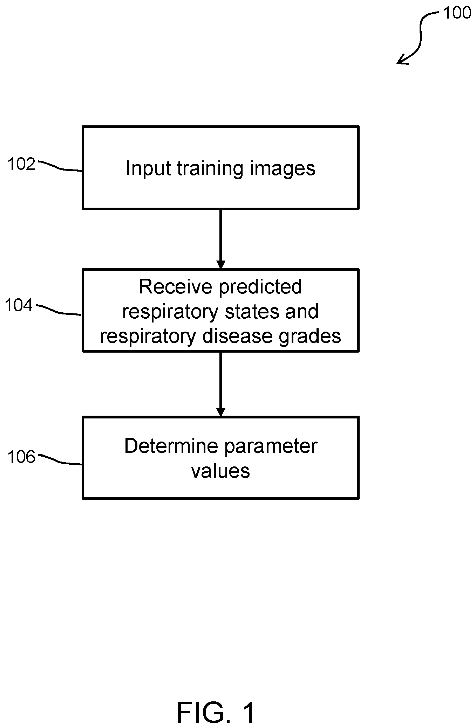

Referring now to Fig. 1, there is depicted a flow diagram of an exemplary embodiment of a method 100 of generating a DAX model. Specifically, the DAX model of this embodiment is adapted to represent relations between a DAX signal, a respiratory disease grade and a respiration state.

In step 102, a plurality of training DAX images of subjects at a plurality of known respiratory states, and with a plurality of known respiratory disease grades are inputted to the DAX model. The training images may include training DAX images corresponding to each of a plurality of respiratory states and may further include training DAX images corresponding to a plurality of respiratory disease grades for each of the plurality of respiratory states. Generally, the greater the number of training DAX images that are input to the DAX model, the more accurate the model will be at representing relations between a DAX signal, a respiratory disease grade and a respiration state.

In some embodiments, the DAX signal may be an integrated signal over a full lung field of a DAX image. Alternatively, the DAX signal may be an integrated signal over part of the full lung field of the DAX image, such as the left lung, right lung, lung apices, or even a single pixel of the lung field.

Further, the training DAX images may be acquired from a known database of DAX images of a plurality of subjects, or may be acquired directly from a plurality of subjects. The respiratory state of the subject during acquisition of the training DAX images may be known due to an external monitoring system. The respiratory disease of the subject may be known by medical records or may be reported by the subject. The training DAX images may be DAX images of a plurality of subject's chests.

At step 104, a plurality of predicted respiratory states and predicted respiratory disease grades from the DAX model are received from the DAX model. In other words, the predicted respiratory states and predicted respiratory disease grades are produced by the DAX model responsive to inputting the plurality of training DAX images, each predicted respiratory state and predicted respiratory disease grade corresponding to one of the plurality of training DAX images.

At step 106, parameter values of the DAX model are determined based on a difference between the plurality of known respiratory states and the plurality of predicted respiratory states. The parameter values are further determined based on a difference between the plurality of known respiratory disease grades and the plurality of predicted respiratory disease states. In this way, parameter values of the DAX model may be set such that the DAX model may accurately represent relations between a DAX signal, a respiratory state and a respiratory disease grade.

It may be the case that the DAX model uses a statistical method to represent the relations between the DAX signal, the respiratory disease grade, and the respiration state. In some embodiments, linear methods may be used as statistical method, such as multivariate regression of the variables. In alternative embodiments, non-linear methods may be used, such as support vector machines or convolutional neural networks that are capable of modelling different kinds of correlations. In embodiments where there exists strong, non-linear correlation between variables, then manifold embedding may be used. It should be understood that this list is not exhaustive, and any statistical method capable of modelling the relations between multiple variables may be used.

In some embodiments, the DAX model may be adapted to represent relations between the DAX signal, the respiratory disease grade, the respiration state and one or more test subject specific variables. Test subject variables may also have an impact on the change of DAX signal with respiratory state, and therefore by including these variables in the model, a more accurate DAX model may be generated. Moreover, the DAX model uses one of the statistical methods above to represent the relation between the DAX signal, the respiratory disease grade, the respiration state and the one or more test subject specific variables.

In this case, the plurality of training DAX images further comprise training DAX images of subjects with the one or more test subject specific variables. For example, if the test subject specific variables includes a sex, then the plurality of training DAX images may include DAX images of subjects who are male, and DAX images of subjects who are female. By way of further example, if the test subject specific variables include an age, then the plurality of training DAX images may include DAX images of different subjects at different ages. Typically, the more training DAX images that are available to input to the DAX model, the more model parameters can be used.

The one or more test subject specific variables may include a sex, a body size, a body mass index, a lung function test result, a respiratory disease rating, known diseases, or a smoking history. It should be understood that this list is not exhaustive, and any subject related variable that has an impact on the DAX signal may be included in the model.

To paraphrase the above, the DAX model is built using a set of training DAX images at different respiratory states and with subjects of different respiratory disease grades measuring the DAX signal. The DAX signal may be either an integrated signal over the whole lung field or some more local measure down to a single pixel. In some embodiments, multivariate regression is used to estimate the specific impact of the respiratory state on the DAX signal. In particular, the impact on the DAX signal by a deviation of the respiratory state from a desired protocol respiration state may be modelled. It may be advantageous to include other variables in the DAX model, as long as they have a specific impact on the DAX signal, there are enough training DAX images available for these variables to be built into the model, and their value can be determined or estimated after training DAX image acquisition.

Overall, the DAX model may enable a prediction of the DAX signal given a respiratory disease grade, a respiratory state, and any other subject specific variables. In some embodiments, the DAX model may also enable a staging of a respiratory disease grade given a DAX signal, a respiratory state, and any other subejct specific variables. Specifically, the DAX model may enable an estimation of the impact of a deviation in respiratory state from a reference respiratory state on a respiratory disease grade or a DAX signal respectively (given values of the subject specific variables are known). In other words, this information may be used to compensate for the impact of the respiratory state, and to estimate a respiratory disease grade or DAX signal for a given acquisition as it would be at a reference respiratory state instead of the observed respiratory state.

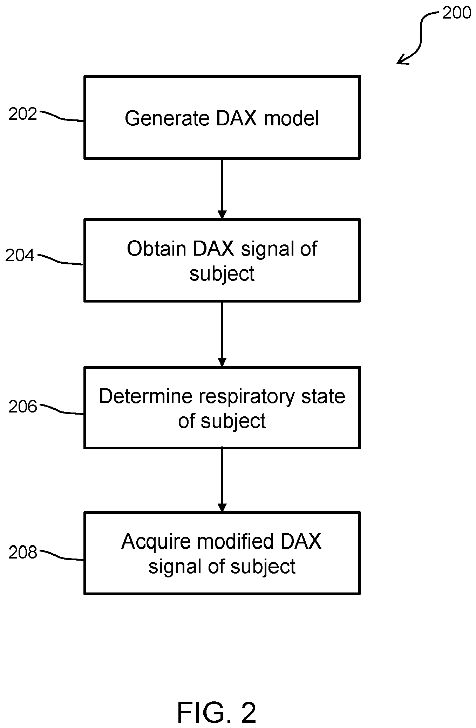

A flow diagram of a method 200 of processing a DAX signal of a subject according to an exemplary embodiment is depicted in Fig. 2.

In step 202, a DAX model is generated. The DAX model may be generated according to any method described in relation to Fig. 1. Nevertheless, the DAX model will be at least adapted to represent the relations between a DAX signal, a respiratory state, and a respiratory diseasegrade.

In step 204, a DAX signal of a subject is obtained. The DAX signal of the subject may be obtained directly from an acquisition session or may be obtained from memory. In any case, the DAX signal will correspond to a DAX image. The DAX image may be obtained from the DAX signal, or vice versa, by any method known to a person skilled in the art.

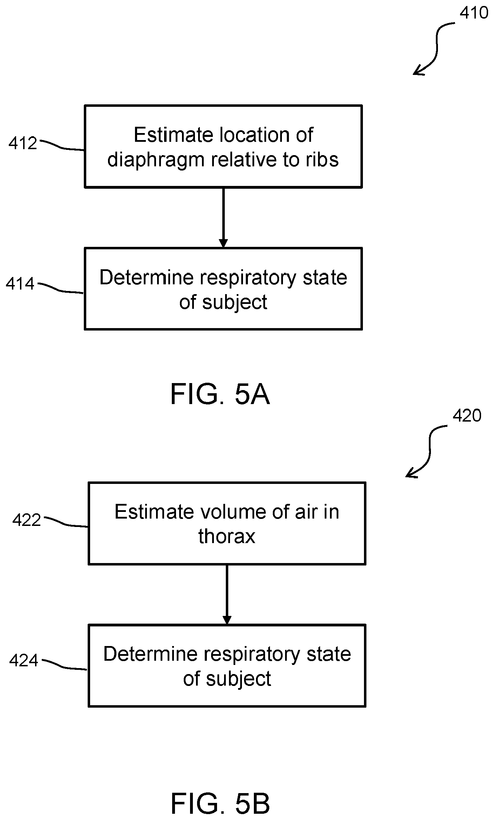

In step 206, a respiratory state of the subject is determined based on the DAX image. It is important that the respiratory state is consistently determined after the acquisition of the DAX image. A number of different methods may perform this. By way of example, spirometry may be used for this purpose, and in many cases, spirometry is part of the diagnostic protocol anyway. Also, external equipment is often used to track breathing, which may include optical or mechanical devices. However, the most suitable method in terms of consistency across subjects, as well as cost, is an estimation of the respiratory/inhalation state based on the DAX image. Methods for determining the respiratory state of the subject will be explained in more detail in reference to Figs. 5A and 5B.

Finally, in step 208, a modified DAX signal of the subject at a reference respiration state is acquired based on inputting the DAX signal and the determined respiratory state to the generated DAX model. The reference respiratory state may be a full inhalation, or a full exhalation state. As the DAX model represents relations between a DAX signal and a respiratory state, if the DAX signal is input to the DAX model, a modified DAX signal at the reference respiratory state may be acquired.

That is to say, the difference in the DAX signal due to a difference between the determined respiratory state and the reference respiratory state may be acquired from the DAX model. As a result, a modified DAX signal may be acquired which corresponds to the reference respiratory state.

Typically, chest X-rays are taken while the subject holds their breath at a full inhalation, or a full exhalation state. This inhalation/exhalation state may be known as the reference respiration state. As some subjects may be unable to hold their breath for the time it takes to acquire the DAX signal, the DAX signal may not correspond to the reference respiration state. It has been shown that this has an impact on the DAX signal, and therefore may lead to inaccurate or wholly incorrect diagnosis. Therefore, this method may provide the advantage of modifying the DAX signal, such that DAX signals are standardized to a reference respiratory state for subsequent assessment.

Alternatively, the DAX model may not be generated. Instead, the DAX model may be stored in memory, and obtained when the method is performed. In such a case, step 202 is omitted. However, the DAX model is the same as the DAX model generated according to methods described in relation to Fig. 1. Thereby, the DAX model is adapted to represent relations between a DAX signal, a respiratory disease grade and a respiration state.

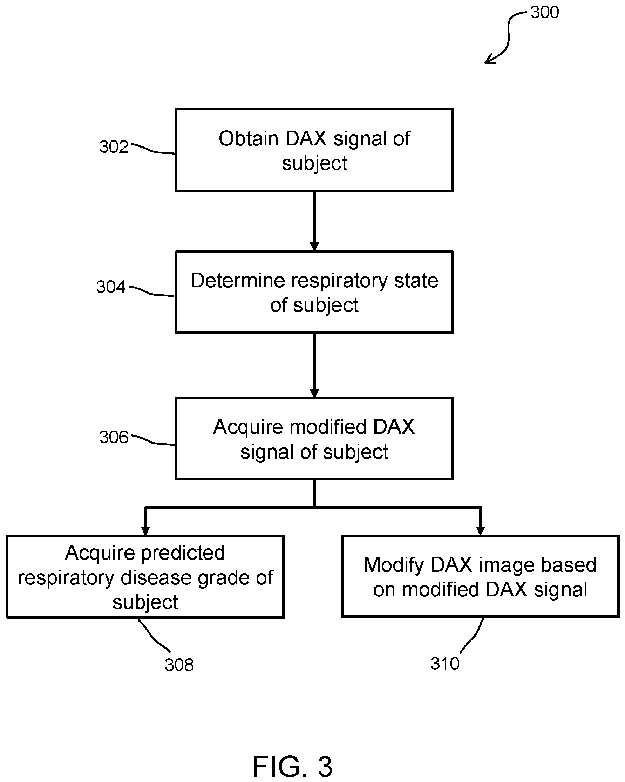

Fig. 3 depicts a flow diagram of another exemplary embodiment of a method 300 of processing a DAX signal of a subject using the DAX model. Steps 302, 304 and 306 of Fig. 3 are equivalent to steps 204, 206 and 208 of Fig. 2, respectively. Thus, explanation of these steps is omitted for the sake of conciseness.

Further, a method 200, 300 of processing a DAX signal of a subject may further comprise step 308 and step 310. In step 308 a predicted respiratory disease grade of the subject is acquired based on inputting the DAX signal and the determined respiratory state to the DAX model. As a result, staging of a respiratory disease in a subject may be performed automatically, aiding in the diagnostic process. As the DAX model represents relations between a respiratory state, a DAX signal, and a respiratory disease grade, both the respiratory state, and the DAX signal may be considered in the respiratory disease grade.

In step 310, the DAX image of the subject is modified based on the modified DAX signal. Accordingly, a modified DAX image may be acquired, which may accurately represent the DAX image at the reference respiratory state.

It should be noted that step 308 and step 310 may be provided independent of one another, or may be provided together.

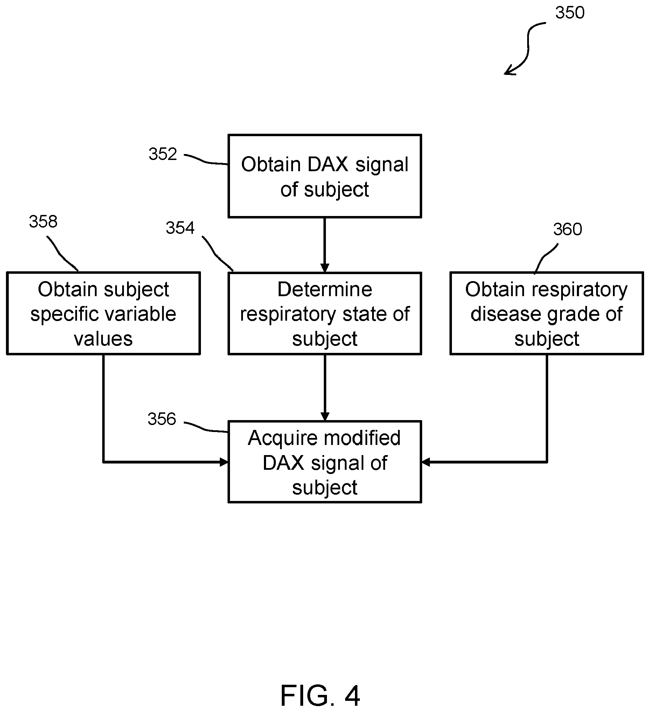

Fig. 4 depicts a flow diagram of an exemplary embodiment of a method 350 of processing a DAX signal of a subject by using a DAX model, and additional data. Specifically, subject specific variable values, and a respiratory disease value are obtained and used to acquire the modified DAX signal of the subject. These additional data may be also comprised in the method 200 of processing a DAX signal. Steps 352 and 354 of Fig. 4 are equivalent to steps 204 and 206 of Fig. 2, respectively. Thus, explanation of these steps is omitted for the sake of conciseness.

At step 358, subject specific variable values of the subject are obtained. The subject specific variable values may include a sex, a body size, a body mass index, a lung function test result, a respiratory disease rating, known diseases, or a smoking history. However, any subject-related variable may be obtained, as long as they have a specific impact on the DAX signal, there are enough training DAX images available for these variables to be built into the model, and their value can be determined or estimated after training DAX image acquisition. In this case, the DAX model is adapted to represent relations between the DAX signal, the respiratory disease grade, the respiration state and one or more subject specific variables. Put another way, the DAX model is further adapted to predict the impact of one or more subject specific variables on the change of DAX signal given a respiratory state, and respiratory disease grade.

Accordingly, when step 358 is provided, acquiring the modified DAX signal of the subject at the reference respiration state (at step 356) is further based on inputting the obtained subject specific variable values to the DAX model, as well as the determined respiratory state of the subject, and the DAX signal. Due to this, the accuracy of the modified DAX signal may be improved.

At step 360, a respiratory disease grade of the subject may be obtained. This may be obtained from a medical record, a separate scan of the subject, or entered by a user. Thus, when step 360 is provided, acquiring the modified DAX signal of the subject at the reference respiration state is further based on inputting the obtained respiratory disease grade to the DAX model. As this adds another fixed variable value to the input of the DAX model, a more accurate modified DAX signal may be obtained.

It is not necessary for step 360 and 358 to be performed together, and only one of these steps may be performed.

Turning to Figs. 5A and 5B, two flow charts depicting a first method 410, and a second method 420 of determining the respiratory state of the subject are provided.

As outlined earlier, it is important that the respiratory state is consistently determined. In many cases, this is performed by spirometry, or external equipment such as optical or mechanical devices. However, the most suitable method in terms of consistency across subjects, as well as cost, is an estimation of the respiratory state based on the DAX image.

Current DAX technology provides both a scatter and a transmission image from the same acquisition. The transmission image (which is very similar to standard chest radiography) is applicable to the both methods described in reference to Figs. 5A and 5B.

In step 412, the location of a diaphragm of the subject relative to ribs of the subject in the DAX image is estimated. In other words, the location of the diaphragm relative to some individual ribs of the subject is determined. Then, in step 414, the respiratory state of the subject is determined based on the estimated location of the diaphragm. Typically, the diaphragm being high compared to the ribs indicates that the subject is close to full exhalation. Conversely, the diaphragm being low compared to the ribs indicates that the subject is close to full inhalation.

In step 422, a volume of air in a thorax of the subject is estimated, based on the lung field of the DAX image. This may be performed by making assumptions about the lung of the subject. Specifically, after replacing rib shadows from the DAX image, the border of the lung field is taken as reference, and intensities along the lung field are integrated. This method may also be used to estimate the lung depth locally at a given image position in the lung field. At step 424, when the volume of air in the thorax has been estimated, the respiratory state of the subject may be determined based on the estimated volume of air.

In some embodiments, only one of the methods described above in reference to Figs. 5A and 5B may be performed. Alternatively, in other embodiments, both methods may be performed, and an average respiratory state of the subject is taken. Accordingly, an accuracy of the determination of the respiratory state may be improved.

In another exemplary embodiment of the present invention, a computer program or a computer program element is provided, which when being executed by at least one processing unit, is adapted to cause the processing unit to perform the steps of any of the methods previously described.

According to a further exemplary embodiment of the present invention, a computer readable medium, such as a CD-ROM, is presented wherein the computer readable medium has a computer program element stored on it which computer program element is described by the preceding section.

A computer program may be stored/distributed on a suitable medium, such as an optical storage medium or a solid-state medium supplied together with or as part of other hardware, but may also be distributed in other forms, such as via the Internet or other wired or wireless telecommunication systems.

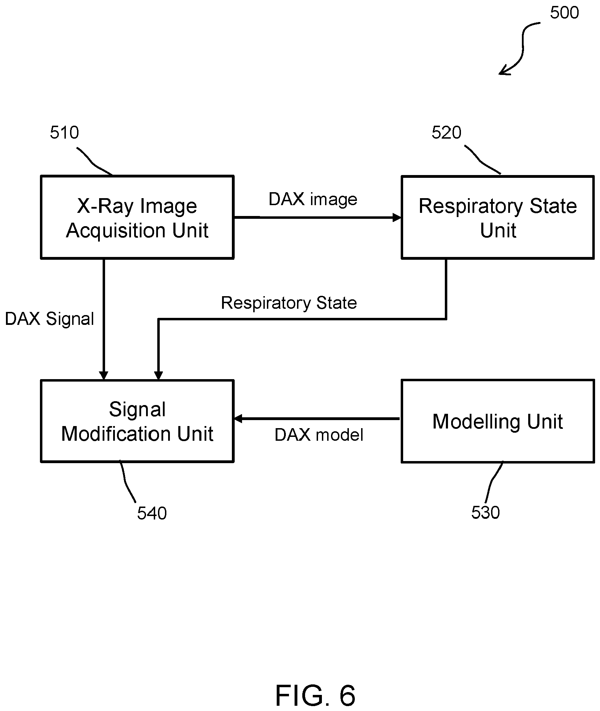

Fig. 6 depicts a simplified block diagram of a system 500 for processing a DAX signal of a subject. The system may be configured to perform any of the methods described above.

Specifically, the system 500 comprises a modelling unit 530 configured to generate a DAX model. The DAX model may be generated by the method 100 described in relation to Fig. 1. Indeed, the DAX model is adapted to represent relations between a DAX signal, a respiratory disease grade and a respiration state.

The system 500 also includes an X-ray image acquisition unit 510, a respiratory state unit 520, and a signal modification unit 540. The X-ray image acquisition unit 510 is configured to acquire the DAX signal of the subject, the DAX signal corresponding to a DAX image. The X-ray image acquisition unit 510 may acquire the DAX signal directly through an image acquisition of the subject (e.g. from an X-ray or CT acquisition session) or may acquire the DAX signal from a database.

The respiratory state unit 520 is configured to determine a respiratory state of the subject based on the DAX image. The respiratory state may be determined according to the methods described in relation to Figs. 5A, or 5B, or may determine the respiratory state through another method.

The signal modification unit 540 is configured to acquire a modified DAX signal of the subject at a reference respiration state based on inputting the DAX signal and the determined respiratory state to the generated DAX model. The signal modification unit may also be adapted to input a respiratory disease grade of the subject to the generated DAX model, or one or more of a plurality of patient specific variable values (if the DAX model is further adapted to represent relations with patient specific variables).

A single processor or other unit may fulfil the functions of several items recited in the claims.

The mere fact that certain measures are recited in mutually different dependent claims does not indicate that a combination of these measures cannot be used to advantage.

Any reference signs in the claims should not be construed as limiting the scope.

## Claims

1. A method (100) of generating a dark-field X-ray, DAX, model adapted to represent relations between a DAX signal, a respiratory disease grade and a respiration state, the method comprising:
inputting (102) a plurality of training DAX images of subjects at a plurality of known respiratory states, and with a plurality of known respiratory disease grades to the DAX model;
receiving (104) a plurality of predicted respiratory states and predicted respiratory disease grades from the DAX model; and
determining (106) parameter values of the DAX model based on a difference between the plurality of known respiratory states and the plurality of predicted respiratory states, and further based on a difference between the plurality of known respiratory disease grades and the plurality of predicted respiratory disease states.

2. The method of claim 1, wherein the DAX model uses a statistical method to represent the relations between the DAX signal, the respiratory disease grade, and the respiration state.

3. The method of any preceding claim, wherein the DAX model is further adapted to represent relations between the DAX signal, the respiratory disease grade, the respiration state and one or more test subject specific variables,
wherein the plurality of training DAX images further comprises training DAX images of subjects with the one or more test subject specific variables, and
wherein the DAX model uses a statistical method to represent the relations between the DAX signal, the respiratory disease grade, the respiration state and the one or more test subject specific variables.

4. The method of claim 3, wherein the one or more test subject specific variables include a sex, a body size, a body mass index, a lung function test result, a respiratory disease rating, known diseases, or a smoking history.

5. The method of any preceding claim, wherein the DAX signal is either an integrated signal over a full lung field of a DAX image, or an integrated signal over part of the full lung field of the DAX image.

6. A method (200) for processing a dark-field X-ray, DAX, signal of a subject, the method comprising:
generating (202) a DAX model according to any of claims 1-5;
obtaining (204) the DAX signal of the subject, wherein a DAX image corresponds to the DAX signal;
determining (206) a respiratory state of the subject based on the DAX image; and
acquiring (208) a modified DAX signal of the subject at a reference respiration state based on inputting the DAX signal and the determined respiratory state to the generated DAX model.

7. A method (300) for processing a dark-field X-ray, DAX, signal of a subject using a DAX model adapted to represent relations between a DAX signal, a respiratory disease grade and a respiration state, the method comprising:
obtaining (302) a DAX signal of the subject, wherein a DAX image corresponds to the DAX signal;
determining (304) a respiratory state of the subject based on the DAX signal; and
acquiring (306) a modified DAX signal of the subject at a reference respiration state based on inputting the DAX signal and the determined respiratory state to the DAX model.

8. The method of claims 6-7, further comprising:
obtaining (360) a respiratory disease grade of the subject, and
wherein acquiring (208, 306) the modified DAX signal of the subject at the reference respiration state is further based on inputting the obtained respiratory disease grade to the DAX model.

9. The method of claims 6, 7 or 8, wherein the DAX model is further adapted to represent relations between the DAX signal, the respiratory disease grade, the respiration state and one or more subject specific variables, and wherein the method further comprises:
obtaining (358) subject specific variable values of the subject, and
wherein acquiring (208, 306) the modified DAX signal of the subject at the reference respiration state is further based on inputting the obtained subject specific variable values to the DAX model.

10. The method of claims 6 to 9, further comprising:
acquiring (308) a predicted respiratory disease grade of the subject based on inputting the DAX signal and the determined respiratory state to the DAX model.

11. The method of claims 6 to 10, wherein the reference respiration state is either a full inhalation state, or a full exhalation state.

12. The method of any of claims 6 to 11, wherein determining the respiratory state of the subject comprises:
estimating (412) a location of a diaphragm of the subject in the DAX image, relative to ribs of the subject in the DAX image; and
determining (414) the respiratory state of the subject is based on the estimated location of the diaphragm.

13. The method of any of claims 6 to 11, wherein determining the respiratory state of the subject comprises:
estimating (422) a volume of air in a thorax of the subject based on a lung field of the DAX image; and
determining (424) the respiratory state of the subject based on the estimated volume of air in the thorax of the subject.

14. The method of any of claims 6 to 13, further comprising:
modifying (310) the DAX image based on the modified DAX signal.

15. A system (500) for processing a dark-field X-ray, DAX, signal of a subject, the system comprising:
a modelling unit (530) configured to generate a DAX model adapted to represent relations between a DAX signal, a respiratory disease grade and a respiration state;
an x-ray image acquisition unit (510) configured to acquire the DAX signal of the subject, wherein a DAX image corresponds to the DAX signal;
a respiratory state unit (520) configured to determine a respiratory state of the subject based on the DAX image; and
a signal modification unit (540) configured to acquire a modified DAX signal of the subject at a reference respiration state based on inputting the DAX signal and the determined respiratory state to the generated DAX model.
